# EUROPEAN PATENT SPECIFICATION

(11) **EP 1 866 308 B1**
(45) Date of publication and mention of the grant of the patent: **22.10.2008**
(21) Application number: 06710423.2
(22) Date of filing: 21.02.2006
(51) Int. Cl.: C07D 471/08

(54) **PREPARATION OF A HIGH PURITY SUBSTITUTED QUINOXALINE**
HERSTELLUNG EINES SUBSTITUIERTEN CHINOXALINS VON HOHER REINHEIT
PREPARATION D'UNE QUINOXALINE SUBSTITUEE DE HAUTE PURETE

(30) Priority: 24.02.2005 US 656296 P
(43) Date of publication of application: 19.12.2007
(73) Proprietor: Pfizer Products Incorporated, Groton, CT 06340 (US)
(72) Inventor: Busch, Frank Robert Pfizer Global Research & Dev., Groton, CT 06340 (US); Hawkins, Joel Michael Pfizer Global Research & Dev, Groton, CT 06340 (US); Mustakis, Lasson, G. Pfizer Global Research & Dev., Groton, CT 06340 (US); Sinay, Terry Gene Jr. Pfizer Global Research & Dev, Groton, CT 06340 (US); Watson, Timothy J. N. Pfizer Global Research & Dev, Groton, CT 06340 (US); Withbroe, Gregory J. Pfizer Global Research & Dev., Groton, CT 06340 (US)
(74) Representative: Hodge, Emma Jane
(86) International application number: PCT/IB2006/000351
(87) International publication number: WO 2006/090236

(56) References cited:
- WO-A-99/35131
- WO-A-02/092089
- WO-A-20/04108725
- US-B1- 6 410 550

## Description

### Background of the Invention

The present invention comprises an improved process for the preparation of substituted quinoxalines by cyclization of the corresponding dianiline. Substituted quinoxalines are useful intermediates in the preparation of aryl fused azapolycyclic compounds.

The synthesis, composition and methods of use of certain aryl fused azapolycyclic compounds are disclosed in United States Patent No. 6,410,550 including the formation of substituted quinoxaline through the cyclization of the corresponding dianiline with aqueous glyoxal. The improvements in product purity of the present invention are needed in order to provide a more cost effective process and a higher quality product of suitable quality for human consumption.

WO 02/092089 discloses preparation of the polymorphs of the L-tartrate salt of the aryl fused azapolycyclic compound having the formula

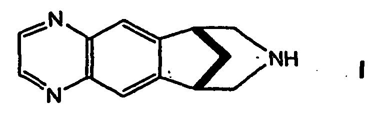

### Summary of the Invention

The process of the present invention provides a method of preparing selected aryl fused azapolycylic compounds with enhanced purity.

The present invention provides a process for preparing a compound containing a chemical moiety of formula **II** 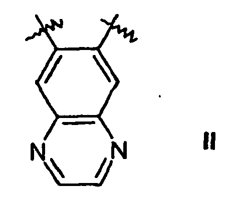 comprising cyclizing a compound containing a chemical moiety of formula **III** 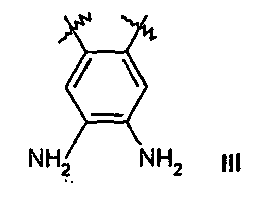 with aqueous glyoxal in a protic solvent.

The compound containing the chemical moiety of formula **III** is prepared by hydrogenating a compound containing a chemical moiety of formula **IV** in a protic solvent In the presence of a solid hydrogenation catalyst.

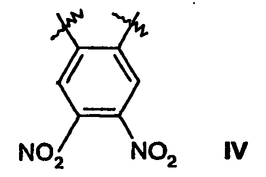

In a preferred embodiment the present invention provides a process for preparing a compound having the formula 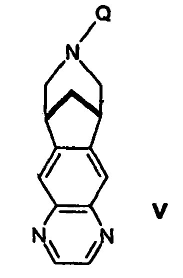 from a compound of the formula 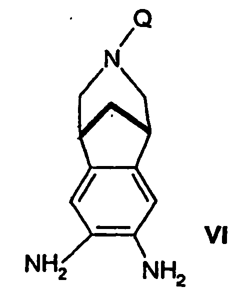 wherein Q is a nitrogen protecting group.

The protic solvent is a water miscible organic solvent including, for example, aliphatic alcohols with one to five carbon atoms, tetrahydrofuran and dimethylformamide, optionally mixed with water. Preferably, the solvent is a C₁-C₅ alcohol. Most preferably, the solvent is comprised of about 80% by weight isopropyl alcohol and about 20% by weight water.

The hydrogenation catalyst is a Group VIII transition metal on a solid support. In a preferred embodiment the catalyst is palladium on carbon, palladium on alumina or palladium on a polymeric support. Most preferably the catalyst is 5% palladium on carbon.

In another preferred embodiment, the aqueous glyoxal is comprised of about 5% to about 20% by weight glyoxal and from about 80% to about 95% by weight water.

In a preferred embodiment, the solid hydrogenation catalyst is present at a level of from about 2% to about 5% by weight of compound of formula **IV;** preferably about 3%.

In a preferred embodiment the solid hydrogenation catalyst is separated from the solution of compound of formula **III** in the protic solvent by filtration.

Q is a nitrogen protecting group. Preferably, Q is a trifluoroacetyl group, an acetyl group or a t-butoxy carbonyl group. Most preferably, Q is a trifluoroacetyl group.

In another embodiment of the present invention, a process is provided for preparing a compound of formula **I** or its pharmaceutically acceptable salt thereof comprising the steps of:
a) reducing a dinitro compound of the formula 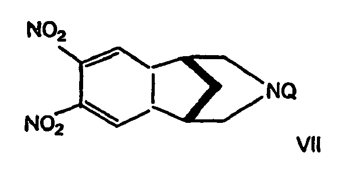 with hydrogen in the presence of a hydrogenation catalyst under conditions effective to form the corresponding dianiline compound, wherein Q is a nitrogen protecting group;
(b) cyclizing the dianiline compound formed in step (a) with aqueous glyoxal to form the corresponding quinoxaline;
(c) removing the nitrogen protecting group Q by hydrolysis of the quinoxaline formed in step (b) with a base in a non-chlorinated solvent; and
(d) isolating the compound of formula **I** from the product of step c as the free base; or, optionally as a pharmaceutically acceptable salt;
wherein steps (a) and (b) are conducted in a protic solvent.

The protic solvent in steps (a) and (b) is a water-miscible organic solvent optionally mixed with water. Preferably the solvent is a C₁-C₅ alcohol. Most preferably, the solvent is comprised of about 80% by weight isopropyl alcohol and about 20% by weight water.

The hydrogenation catalyst is a Group **VIII** transition metal on a solid support. In a preferred embodiment the catalyst is comprised of palladium on carbon, palladium on alumina or palladium on a polymeric support. Most preferably the catalyst is 5% palladium on carbon.

In a preferred embodiment, the cyclization step (b above) is conducted at a temperature range of from about -10°C to about 20°C; most preferably, the temperature is about 0°C to about 15°C and the aqueous glyoxal is comprised of about 5% to about 15% by weight glyoxal and from about 85% to about 95% by weight water. The pH is maintained at a range of from about 6 to about 8.

In another embodiment of the present invention, the cyclization step (b above) is conducted under basic conditions by controlling the pH above about 7 through the presence of a buffering agent in the reaction mixture or through dosing in a solution of a suitable base. Suitable buffering agents include, but are not limited to salts of Group I and II metal bases and a weak acid. Preferred buffering agents include NaHCO₃, Na₂CO₃ and mixtures of Na₂HPO₄ and NaH₂PO₄; most preferably the buffering agent is NaHCO₃. Preferably the buffering agent is present in the amount of from about 0.005 equivalents to about 0.20 equivalents; most preferably in the amount of about 0.01 equivalents. Alternatively, the buffer as used in the invention may in some cases be supplied by selection of water which is naturally high In buffer, such as hard water from a well in a locality naturally high in calcium carbonate deposits or the like.

In a preferred embodiment, the nitrogen protecting group Q is the trifluoroacetyl goup.

In step (d) the preferred salt is the L-tartrate salt.

In step (c) the non-chlorinated solvent is preferably toluene and the base is preferably sodium hydroxide.

The invention also relates to a compound of the formula **V** containing a maximum of about 500 ppm of a compound of formula **VIII.**

For convenience, certain terms employed* in the specification, examples and appendant claims are collected here. Unless defined otherwise, all technical and scientific terms used herein have the same meaning as commonly understood by one of ordinary skill in the art to which this invention belongs.

The term "cyclizing", as used herein refers to a chemical reaction in which a linear or branched chemical moiety or a substituted ring moiety is converted into a new ring moiety.

The term "buffering agent " as used herein refers to a chemical compound or mixture of chemical compounds which, when dissolved in an appropriate solvent such as water, provide a solution containing both a weak acid and its conjugate base, wherein the pH of said solution changes only slightly on addition of add or base.

### Detailed Description of the Invention

Scheme 1 below illustrates a specific example of the present invention whereby the aryl fused azapolycyclic compound of formula **I** is prepared in high purity and yield.

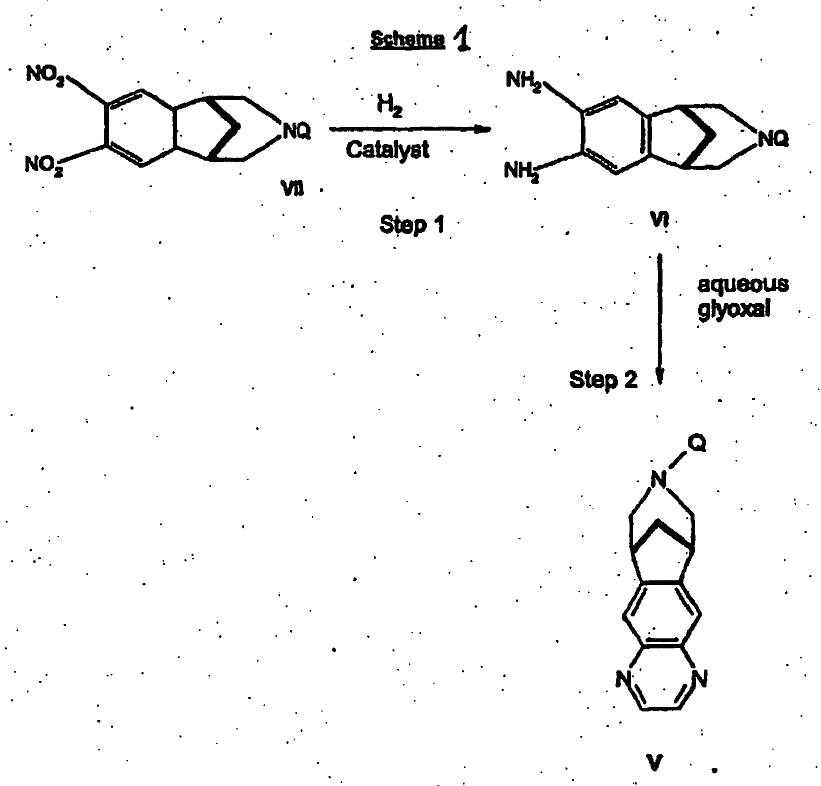

Prior attempts, as disclosed in United States Patent No. 6,410,550, to convert the compounds of formula **VII** into the compound of formula **V** utilized either 40% aqueous glyoxal or the addition adduct of sodium bisulfite and ethane dione. Both of these reactions required certain purification steps.

The present inventors have discovered, quite surprisingly, that the overall purity of the final product obtained from the sequence of steps illustrated in Scheme **1** can be significantly increased by incorporating the new reaction conditions and procedures described herein below.

Additional examples of selected reaction conditions employed in the present invention which provide increased overall purity of the final product obtained according to Scheme **1** include:
1. Dilution of 40% aqueous glyoxal to a concentration of from about 5% to about 20% in Scheme **1**.
2. Maintaining a pH above 7 in the cyclization step through the addition of a buffering agent.
3. A reaction temperature in Scheme **1** selected from the range of from about -10°C to about 20°C.
4. Effective removal of hydrogenation catalyst by filtration prior to cyclization step in Scheme **1**.

As illustrated in Scheme **2** below, compounds of formula **VII** are precursors to the aryl fused azapolycyclic compound of formula **I** and its pharmaceutically acceptable acid salts. Preferably the acid salt is the L-tartaric acid salt.

The compound of formula **I** is useful in the treatment of central nervous system disorders as described herein.

Removal of the nitrogen protecting group Q is carried out by methods well known in the art, such as, heating with base in a solvent mixture of water and a water immiscible organic solvent including, for example, toluene or methylene chloride. Most preferably, the base is sodium hydroxide and the organic solvent is toluene.

Optionally, colored impurities are removed by treating a methanolic solution of V**II** with activated carbon. 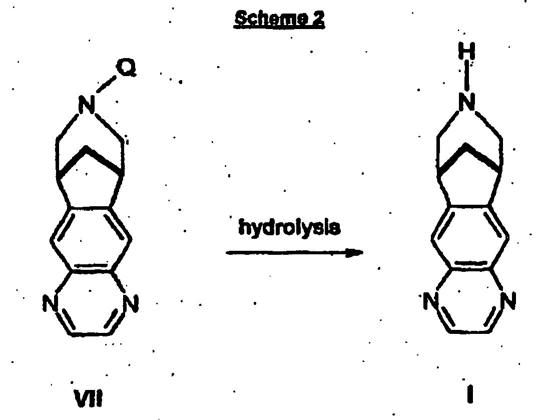 wherein Q is as defined above.

In the practice of the present invention according to Scheme **1,** a compound of formula bristle V**III** is formed. Without being bound to a particular theory, it is believed that the formation of V**III** is minimized by avoiding side reactions in Step **2** of Scheme **1**.

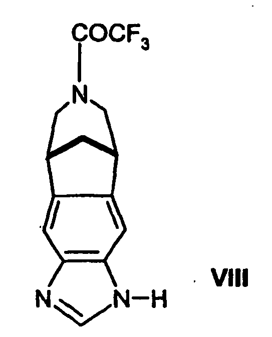

Thus, isopropyl alcohol is preferred over methanol. Furthermore, certain forms of carbon should be avoided. Examples of undesirable forms of carbon include but are not limited to carbon supports used in hydrogenation catalysts and activated carbon normally used as a purification and decolorizing agent.

Compounds of formula **I** and its pharmaceutically acceptable salts bind to neuronal nicotinic acetylcholine specific receptor sites and are useful in modulating cholinergic function. Such compounds are useful in the treatment of inflammatory bowel disease (including but not limited to ulcerative colitis, pyoderma gangrenosum and Crohn's_disease), irritable bowel syndrome, spastic dystonia, chronic pain, acute pain, celiac sprue, pouchitis, vasoconstriction, anxiety, panic disorder, depression, bipolar disorder, autism, sleep disorders, jet lag, amyotrophic lateral sclerosis (ALS), cognitive dysfunction, hypertension, bulimia, anorexia, obesity, cardiac arrythmias, gastric add hypersecretion, ulcers, pheochromocytoma, progressive supranuclear palsy, chemical dependencies and addictions (e.g., dependencies on, or addictions to nicotine (and/or tobacco products), alcohol, benzodiazepines, barbiturates, opioids or cocaine), headache, migraine, stroke, traumatic brain injury (TBI), obsessive-compulsive disorder (OCD), psychosis, Huntington's chorea, tardive dyskinesia, hyperkinesia, dyslexia, schizophrenia, multi-infarct dementia, age-related cognitive decline, epilepsy, including petit mal absence epilepsy, senile dementia of the Alzheimer's type (AD), Parkinson's disease (PD), attention deficit hyperactivity disorder (ADHD) and Tourette's Syndrome.

The following examples are provided for the purpose of further illustration and are not intended to limit the scope of the claimed invention.

### EXAMPLE 1

### Cyclization of 2,3,4,5-tetrahydro-3-(trifluoroacetyl)-1,5-methano-1H-3-benzazepine-7,8-diamine (compound VI) with glyoxal to form 7,8,9,10-tetrahydro-8-(trifluoroacetyl)-6,10-methano-6H-pyrazino[2,3-h][3]benzazepine (compound V).

A 600cc Parr Hastelloy Reactor was charged under nitrogen with 10g (28.9 mmol) of compound **VII**, 3%/wt. 300 mg of 5% Pd/C 50% water wet and 200ml (20 vols) of a IPO/Water (80/20) mixture. The reaction was placed under 45-50 psi of hydrogen at 28-30°C for 4 hours. Analysis by HPLC confirmed the reaction to compound **VI** was complete. The reaction was filtered over Celite, a 0.45 micron Millipore Filter and washed with 10 ml of IPO. The light yellow solution of compound **VI** was cooled to 0-5°C and 4.49 g (30.9 mmol) 1.07 eq. of fresh 40% aqueous glyoxal (Aldrich ) diluted with 20 ml of water (8% solution) was added drop wise over a **1** hour period while maintaining 0-5°C. The light orange reaction (pH = 6.9) was stirred for an additional 2 hours at 0-5°C and 20°C for 18 hrs. (lighter in color). Analysis by HPLC confirmed the reaction to compound V was complete. The reaction was vacuum concentrated to a volume of 85 ml (8.5 vols) in a 40-45°C water bath. The concentrate was cooled to 20-23°C and 140 ml of water was added drop wise over 1-1.5 hours. The off-white suspension was granulated for 2 hours at 20-23°C, filtered over a cotton cloth and washed with 10 ml of water. Vacuum dried at 45°C for 16-20 hours. This yielded 7.52 g (84.5%) of an off-white to white solid compound of formula **V** having a purity of 99.9% and a potency of 100.3% as compared to a standard.

### EXAMPLE 2

### Example 1 repeated under acidic conditions

The procedure in Example **1** was repeated with the addition of (0.1 eq) 0.07 ml of phosphoric acid dissolved in the aqueous glyoxal. The reaction mixture had an initial pH of 0.5.

Upon completion of the cyclization, the reaction mixture was analyzed by HPLC showing a 16.1 % formation of compound **VIII.**

### EXAMPLE 3

### Example 1 repeated under basic coniditions

The procedure in Example 1 was repated with the addition of (0.1 eg) 3.1 ml of 1N sodium hydroxide dissolved in the aqueous glyoxal. The reaction mixture had a pH of 9.8.

Upon completion of the cyclization, the reaction mixture was analyzed by HPLC showing a non-detectable level of compound **VIII,** but with two unknown impurities.

### EXAMPLE 4

### Deprotection of 7,8,9,10-tetrahydro-8-(trifluoroacetyl)-6,10-methano-6H-pyrazino[2,3-h][3]benzazepine (compound V) to form 7,8,9,10-tetrahydro-6,10-methano-6H-pyrazino[2,3-h][3]benzazepine (compound I)

### The Hydrolytic Conversion Of Compound VII To The Tartrate Salt of Compound I

### A. Toluene

Compound of formula **V** was slurried in a 2M NaOH solution (3.1 equiv.). Toluene (7 volumes ) was added and the biphasic slurry warmed to 37-40°C. The resulting yellow/brown biphasic mixture was maintained with stirring at 37-40°C for 2-3 hours. Once reaction was complete, an additional 10 volumes toluene was added, and the mixture stirred for 30 min. The biphasic system was taken off stir and the layers allowed to separate. The aqueous layer was collected, extracted with toluene (5 volumes), and the toluene layers combined.

The pot was vacuum distilled to 5 volumes. Methanol (15 volumes) was added, and the pot azeotropically distilled under vacuum to 5 volumes. Methanol (10 volumes) was added and again the pot azeotropically distilled under vacuum to 5 volumes. The resulting methanolic solution of compound **I** was diluted with additional methanol (15 volumes), treated with Darco KB-B (10% w/w) for 1 hour (to remove color), filtered, and transferred to an addition vessel.

In a separate vessel, L-(+)-tartaric acid (1.1 equiv.) was dissolved In methol (13 volumes). The compound of formula I/MeOH solution was then added dropwise to the L-(+)-tartaric acid/MeOH solution. The resulting slurry was allowed to granulate for a minimum of 1 hour, filtered, rinsed with methanol, and allowed to dry under vacuum at 45°C.

### B. Chlorinated Solvent

Compound of formula **V** was slurried in a 1M NaOH solution (3.1 equiv.). Methylene chloride (2.5 volumes) is added and the biphasic mixture stirred for 3-4 hours at room temperature. Once reaction was complete an additional 7.5 volumes methylene chloride was added and the mixture stirred for 30 min. The biphasic system was taken off stir and the layers allowed to separate. The aqueous layer was collected, extracted with methylene chloride (5 volumes), and the methylene chloride layers combined.

The pot was atmospherically distilled to 5 volumes. Methanol (10 volumes) was added, and the pot distilled under partial vacuum to 5 volumes. Methanol (5 volumes) was added, and the pot distilled under full vacuum to 5 volumes. The resulting compound of formula I/MeOH solution was diluted with additional methanol (18 volumes), treated with Darco KB-B (10% w/w) for 1 hour (to remove color), filtered, and transferred to an addition vessel.

In a separate vessel, L-(+)-tartaric acid (1.1 equiv.) was dissolved in methanol (13 volumes). The Compound I/MeOH solution was then added dropwise to the L-(+)-tartaric acid/MeOH solution. The resulting slurry was allowed to granulate for a minimum of 1 hour, filtered, rinsed with methanol, and allowed to dry under vacuum at 45°C.

### C. UV-Absorbance Comparison of Laboratory Generated Compound I, Tartrate Salt

Starting from the same lot of compound of formula V, the tartrate salt of compound **I** was synthesized via both the toluene and methylene chloride processes. See the Table below for results summary:

| Absorbance Of Compound I Tartrate Salt Solutions | | | |
|---|---|---|---|
| Procedure | Conditions | Darco * | Absorbance at 430nm |
| B | CH₂Cl₂ | Yes | 2.0 |
| A | Toluene | No | 8.1 |
| A | Toluene | Yes | 2.1 |

| | | | |
|---|---|---|---|
| Darco KB-B brand activated carbon | | | |

As the Table indicates, both the CH₂Cl₂ and toluene processes, where Darco KB-B^{™} was used, gave compound of formula I tartrate salt of comparable quality. This Table further demonstrates how effective the Darco KB-B^{™} was at removing color from the toluene process (UV-abs of 2.1 when Darco KB-B^{™} was used vs. 8.1 when Darco KB-B^{™} was not used, nearly a 4x difference).

### EXAMPLE 5

### Reprocessing Conditions

Compound of formula **I** tartrate salt was dissolved in water (5 volumes), followed by addition of toluene (10 volumes). A 50% NaOH (aq) solution (2.2 equiv.) was added until the pH = 12-13 (this was approximately 0.5 volumes of the NaOH (aq) solution). A particular lot was chosen as the starting material for the rework due to its color being more yellow than typical.

After stirring for 1.5 hours at 37-40°C, the layers were allowed to separate. The toluene layer was collected and set aside. The aqueous layer was extracted with toluene (5 volumes), the toluene layers combined and vacuum distilled to 5 volumes. Methanol (15 volumes) was added and the pot azeotropically distilled under vacuum to 5 volumes. Methanol (10 volumes) was added, and again, the pot azeotropically distilled to 5 volumes.

The resulting compound of formula I/MeOH solution was diluted with methanol (18 volumes). Darco KB-B^{™} (10% w/w) was added, stirred for one hour and filtered through a pad of celite. The methanolic solution of Compound **I** was then transferred to an addition funnel. In a separate vessel, L-(+)-tartaric add (1.1 equiv) was dissolved in methanol (13.5 volumes). The compound of formula I/MeOH solution was then added dropwise to the L-(+)-tartaric acid/MeOH solution. The resulting slurry was allowed to granulate for a minimum of 1 hour, filtered and dried. This gave an 80% recovery of compound of formula **I** tartrate salt as a white solid.

### EXAMPLE 6

The formation of compound of formula **VIII** is an indication of the side reactions which are occurring, and is not the only impurity peak observed in the assay of these materials. Several of the additional impurities have not been identified; however, the identification was not necessary as the impurities are controlled when the formation of compound of formula **VIII** is controlled. Thus, compound of formula **VIII** is valuable as a marker showing that the process has been run in a manner to minimize the amount of impurities which may form.

Table 1 summarizes comparative data of two pilot plant production lots using conditions of the present invention with data obtained from two pilot plant runs using "old process" conditions of the prior art.

**Table 1: Summary of data on Compound VIII**

| Lot | Compound VIII | Reaction. Conditions |
|---|---|---|
| Lot 1 | 4800 ppm | Old Process |
| Lot 2 | 1700 ppm | Old Process |
| | | |
| Lot 3 | 500 ppm | Present Invention |
| Lot 4 | ND* | Present Invention |

| | | |
|---|---|---|
| *ND = not detected at limit of quantitation (<500ppm) | | |

Thus, lower levels of compound of formula **VIII** and other unidentified compounds, are present in compound of formula V when the process is properly controlled according to the teachings of the present invention. As shown In Table 1, the "old", process resulted in 4800 to 1700 ppm of the compound of formula **VIII,** while the present process resulted in dramatically reduced levels of 500 ppm or less.

### EXAMPLE 7 (Buffering Agent)

### Cyclization of 2,3,4,5-tetrahydro-3-(trifluoroacetyl)-1,5-methano-1H-3-benzazepine-7,8-diamine (compound VI) with glyoxal to form 7,8,9,10-tetrahydro-8-(trifluoroacetyl)-6,10-methano-6H-pyrazino[2,3-h][3]benzazepine (compound V).

A 600 cc Parr Hastelloy B Reactor was charged under nitrogen with 10 g (28.9 mmol) of compound **VII**, 3%/wt. 300 mg of 5%Pd/C 50% water wet and 200 ml (20 vols) of a IPO/ Deionized Water (80/20) mixture. The reaction was placed under 20 psi of hydrogen at 20-22°C for 30 minutes, 20 psi for 30 min for 23-25°C and 44 psi for 4 hours at 23-26 °C. The reaction was filtered 26-28°C over Celite, a 0.45 micron Millipore Filter and washed with 40 ml of IPO (total volume= 250 ml, pH=9.6). To this light yellow solution of compound **VI** was added 0.51g (0.1eq) of dibasic potassium phosphate, cooled to 3-6°C and stirred at an RPM = 450. When a temperature of 7°C was obtained a thick suspension appeared. To this suspension at 3-6°C (pH=9.6) was added a solution of 4.49g (30.9 mmol) 1.07 eq. of 40% aqueous Glyoxal diluted with 16 ml of deionized water (8.9% solution, pH=2.95) drop-wise over a 1 hour period (total volume = 268 ml). The light orange reaction was stirred for an additional 2 hours at 3-6°C (pH=9.5) and then allowed to warm to 20-22°C for 18 hrs. (light yellow in color, pH= 9.3). Analysis by HPLC confirmed the reaction to compound **V** was complete and a level of 0.06% compound **VIII** was observed. The reaction was vacuum concentrated to a volume of 75 ml (7.5vols) in a 40-45°C water-bath. The concentrate was cooled to 20-23°C and 140 ml of deionized water was added dropwise over 1 hour (K.F.= 82%). The off-white suspension was granulated for 4 hours at 20-23°C, filtered over a paper filter and washed with 20 ml of deionized water. Vacuum dried at 45°C for 16-20 hours. This yielded 7.85 g (882 %) of an off-white solid.

### EXAMPLE 8 (Buffering Agent)

### Cyclization of 2,3,4,5-tetrahyrdro-3-(trifluoroacetyl)-1,5-methano-1H-3-benzazepine-7,8-diamine (compound VI) with glyoxal to form 7,8,9,10-tetrahydro-8-(trifluoroacetyl)-6,10-methano-6H-pyrazino[2,3-h][3]benzazepine (compound V)..

The Parr Hastelloy B Reactor from Example 7 was charged with reagents following the example of Example 7 above, but with the addition of 121.8 mg (0.05eq) of sodium bicarbonate to the filtered compound **VI** solution (pH=9.6). The procedure was repeated with 4.49 g (30.9 mmol) 1.07 eq. of 40% aqueous Glyoxal diluted with 16 ml of deionized water (8.9% solution, pH=2.85) again added dropwise over a 60 minute period (total volume = 268ml). The light orange reaction was stirred for an additional 2 hours at 3-6°C (pH=9.4) and then allowed to warm to 20-22°C for 18 hrs. (light yellow in color, pH= 9.4). Analysis by HPLC confirmed the reaction to compound V was complete and a level of 0.06% compound **VIII** was observed. The reaction was vacuum concentrated to a volume of 75 ml (7.5 vols) in a 40-45°C water-bath. The concentrate was cooled to 20-23°C and 140 ml of deionized water was added dropwise over 1 hour (K.F.= 81%, pH=9.3). The off-white suspension was granulated for 4 hours at 20-23°C, filtered over a paper filter and washed with 20 ml of deionized water. Vacuum dried at 45°C for 16-20 hours. This yielded 7.85 g (82.0 %) of an off-white solid.

## Claims

1. A process for preparing a compound having the structure: 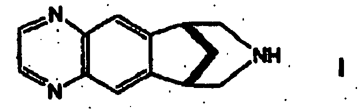 comprising (a) cyclizing a compound having the structure: 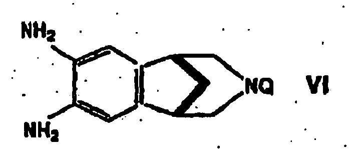 wherein Q is a nitrogen protecting group, with aqueous glyoxal in a protic solvent, to form the corresponding quinoxaline; and,
(b) removing the nitrogen protecting group Q by hydrolysis of the quinoxaline formed in step (a) with a base in a non-chlorinated solvent.

2. The process of claim 1 wherein said compound having structure VI is prepared by hydrogenating a compound having the structure VII: 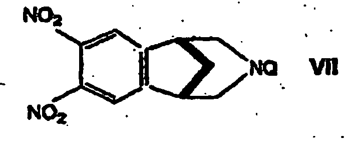 in a protic solvent in the presence of a solid hydrogenation catalyst.

3. The process as in claim 1 or 2, wherein said protic solvent is a water-miscible organic solvent, optionally mixed with water.

4. The process as in claim 3 wherein said water-miscible organic solvent is a C₁-C₅ alcohol.

5. The process according to claim 2 wherein said hydrogenation catalyst is a Group VIII transition metal on a solid support.

6. The process according to claim 5 wherein said Group VIII transition metal catalyst is comprised of palladium on a solid support, said solid support being selected from the group consisting of carbon, alumina and a polymer.

7. The process according to claim 1 wherein said cyclization is conducted at a temperature in the range of about -10°C to about 20°C.

8. The process according to claim 1 wherein said cyclization is conducted under basic conditions by maintaining a pH above about 7 through the presence of a buffering agent in the reaction mixture or through dosing in a solution of a suitable base.

9. The process according to claim 8 wherein said buffering agent is a salt of a Group I or II metal base and a weak add.

10. The process according to claim 9 wherein said buffering agent is NaHCO₃, Na₂CO₃, a mixture of Na₂HPO₄ and NaH₂PO₄, KHCO₃, K₂CO₃ or a mixture of K₂HPO₄ and KH₂PO₄.

11. The process according to claim 8 wherein said buffering agent is NaHCO₃.

12. The process according to claim 8 wherein said buffering agent is present in the amount of from about 0.005 equivalents to about 0.20 equivalents.

13. The process according to claim 1 wherein said aqueous glyoxal is comprised of about 5% to about 20% by weight glyoxal and about 80% to about 95% by weight water.

14. The process according to claim 1 further comprising (c) isolating the compound of formula I as the free base; or, optionally as a pharmaceutically acceptable salt.

15. The process according to claim 1 wherein said non-chlorinated solvent is toluene and said base is sodium hydroxide.

## Patentansprüche

1. Verfahren zur Herstellung einer Verbindung mit der Struktur: 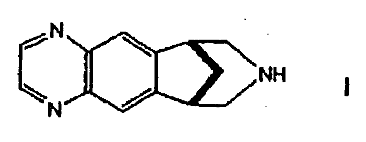 umfassend (a) Cyclisierung einer Verbindung mit der Struktur: 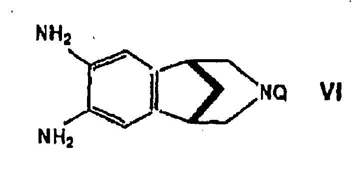 worin Q eine Stickstoffschutzgruppe ist, mit wässrigem Glyoxal in einem protischen Lösungsmittel, um das entsprechende Chinoxalin zu bilden; und
(b) Entfernung der Stickstoffschutzgruppe Q durch Hydrolyse des in Schritt (a) gebildeten Chinoxalins mit einer Base in einem chlorfreien Lösungsmittel.

2. Verfahren nach Anspruch 1, wobei die Verbindung mit der Struktur VI hergestellt wird durch Hydrierung einer Verbindung mit der Struktur VII: 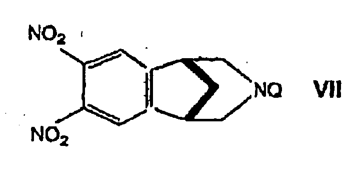 in einem protischen Lösungsmittel in Gegenwart eines festen Hydrierungskatalysators.

3. Verfahren nach Anspruch 1 oder 2, wobei das protische Lösungsmittel ein wassermischbares organisches Lösungsmittel ist, gegebenenfalls im Gemisch mit Wasser.

4. Verfahren nach Anspruch 3, wobei das wassermischbare organische Lösungsmittel ein C₁-C₅-Alkohol ist.

5. Verfahren nach Anspruch 2, wobei der Hydrierungskatalysator ein Gruppe-VIII-Übergangsmetall auf einem festen Träger ist.

6. Verfahren nach Anspruch 5, wobei der Gruppe-VIII-Übergangsmetallkatalysator Palladium auf einem festen Träger umfasst, wobei der feste Träger ausgewählt ist aus der Gruppe, bestehend aus Kohlenstoff, Aluminiumoxid und einem Polymer.

7. Verfahren nach Anspruch 1, wobei die Cyclisierung durchgeführt wird bei einer Temperatur im Bereich von etwa -10°C bis etwa 20°C.

8. Verfahren nach Anspruch 1, wobei die Cyclisierung durchgeführt wird unter basischen Bedingungen durch Beibehaltung eines pHs oberhalb etwa 7 durch die Anwesenheit einer Puffersubstanz in dem Reaktionsgemisch oder durch.Zudosieren einer Lösung einer geeigneten Base.

9. Verfahren nach Anspruch 8, wobei die Puffersubstanz ein Salz einer Gruppe-I- oder -II-Metallbase und einer schwachen Säure ist.

10. Verfahren nach Anspruch. 9, wobei die Puffersubstanz NaHCO₃, Na₂CO₃, ein Gemisch aus Na₂HPO₄ und NaH₂PO₄, KHCO₃, K₂CO₃ oder ein Gemisch aus K₂HPO₄ und KH₂PO₄ ist.

11. Verfahren nach Anspruch 8, wobei die Puffersubstanz NaHCO₃ ist.

12. Verfahren nach Anspruch 8, wobei die Puffersubstanz in einer Menge von etwa 0,005 Äquivalenten bis etwa 0,20 Äquivalenten vorliegt.

13. Verfahren nach Anspruch 1, wobei das wässrige Glyoxal etwa 5 Gew.-% bis etwa 20 Gew.-% Glyoxal und etwa 80 Gew.-% bis etwa 95 Gew.-% Wasser umfasst.

14. Verfahren nach Anspruch 1, des Weiteren umfassend (c) Isolierung der Verbindung der Formel I als freie Base oder gegebenenfalls als ein pharmazeutisch verträgliches Salz.

15. Verfahren nach Anspruch 1, wobei das chlorfreie Lösungsmittel Toluol und die Base Natriumhydroxid ist.

## Revendications

1. Procédé de préparation d'un composé ayant la structure suivante : 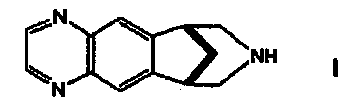 et comprenant :
(a) la cyclisation d'un composé ayant la structure suivante : 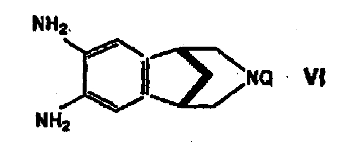 où Q est un groupe protecteur de l'azote, avec du glyoxal aqueux dans un solvant protique, pour former la quinoxaline correspondante ; et
(b) l'élimination du groupe protecteur de l'azote Q par hydrolyse de la quinoxaline formée à l'étape (a), avec une base, dans un solvant non-chloré.

2. Procédé selon la revendication 1, dans lequel ledit composé ayant la structure VI est préparé par hydrogénation d'un composé ayant la structure VII : 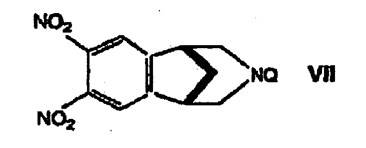 dans un solvant protique, en présence d'un catalyseur d'hydrogénation solide.

3. Procédé selon la revendication 1 ou 2, dans lequel ledit solvant protique est un solvant organique miscible à l'eau, facultativement mélangé avec de l'eau.

4. Procédé selon la revendication 3, dans lequel ledit solvant organique miscible à l'eau est un alcool en C₁-C₅.

5. Procédé selon la revendication 2, dans lequel ledit catalyseur d'hydrogénation est un métal de transition du groupe VIII sur un support solide.

6. Procédé selon la revendication 5, dans lequel ledit catalyseur métallique de transition du groupe VIII est composé de palladium sur un support solide, ledit support solide étant sélectionné dans le groupe consistant en le carbone, l'alumine et un polymère.

7. Procédé selon la revendication 1, dans lequel ladite cyclisation est mise en oeuvre à une température située dans la gamme allant d'environ -10°C à environ 20°C.

8. Procédé selon la revendication 1, dans lequel ladite cyclisation est mise en oeuvre dans des conditions basiques en maintenant le pH au-dessus d'environ 7 par la présence d'un agent tampon dans le mélange réactionnel ou par respect de proportions dans une solution d'une base convenable.

9. Procédé selon la revendication 8, dans lequel ledit agent tampon est un sel d'une base métallique du groupe I ou II et d'un acide faible.

10. Procédé selon la revendication 9, dans lequel ledit agent tampon est le NaHCO₃, le Na₂CO₃, un mélange de Na₂HPO₄ et de NaH₂PO₄, le KHCO₃, le K₂CO₃ ou un mélange de K₂HPO₄ et de KH₂PO₄.

11. Procédé selon la revendication 8, dans lequel ledit agent tampon est le NaHCO₃.

12. Procédé selon la revendication 8, dans lequel ledit agent tampon est présent en une quantité allant d'environ 0,005 équivalent à environ 0,20 équivalent.

13. Procédé selon la revendication 1, dans lequel ledit glyoxal aqueux est composé d'environ 5 % à environ 20 % en poids de glyoxal et d'environ 80 % à environ 95 % en poids d'eau.

14. Procédé selon la revendication 1, comprenant en outre (c) l'isolement du composé de formule I comme base libre ; ou, facultativement, comme sel pharmaceutiquement acceptable.

15. Procédé selon la revendication 1, dans lequel ledit solvant non-chloré est le toluène et ladite base est l'hydroxyde de sodium.
